# EUROPEAN PATENT APPLICATION

(11) **EP 4 566 551 A1**
(43) Date of publication of application: **11.06.2025**
(21) Application number: 23849570.9
(22) Date of filing: 18.07.2023
(51) Int. Cl.: A61B 17/03, A61B 17/08

(54) **DYNAMIC ABDOMINAL WALL TRACTION SYSTEM FOR OPEN ABDOMEN WOUNDS**

(30) Priority: 01.08.2022 ES 202230710
(71) Applicant: Servicio Cántabro de Salud, 39011 Santander (Cantabria) (ES)
(72) Inventor: CASTILLO SUESCÚN, Federico, 39011 Santander (Cantabria) (ES)
(74) Representative: Herrero & Asociados, S.L.
(86) International application number: PCT/ES2023/070464
(87) International publication number: WO 2024/028531

(57) **Abstract**

**The** invention relates to a dynamic abdominal wall (AW) traction system for open abdomen (OA) wounds, which can be temporarily connected to an OA wound for gradual and controlled closure, the system comprising: elastomeric rings (1), which can be connected in the vicinity of the edges of the OA; traction wires (2), intertwined in the form of a zigzag between the rings (1); a traction element (3) for exerting variable and controllable traction on the traction wires (2) and on the rings (1); and retaining elements (4), which can be connected by suturing to an internal edge of the AW and to each ring (1), such that the rings (1) and the OA wound are joined indirectly by means of the suture for connecting the respective elements.

## Description

### OBJECT OF THE INVENTION

The present invention relates to the technical field of devices for closing wounds, as well as to clamps for wounds, and specifically refers to a dynamic wall traction system for open abdominal wounds.

### BACKGROUND OF THE INVENTION

In cases of surgical interventions within the abdominal cavity that require transcutaneous incisions to access the interior, the primary closure of the abdominal cavity restores the anatomy and physiology of the abdominal wall, providing protection to the abdominal contents and reducing complications of the surgical wound. However, there are certain circumstances (e.g., polytrauma, severe septic abdomen, severe acute pancreatitis) that may result in abdominal compartment syndrome (ACS), where primary closure is not possible, necessitating the performance of a temporary abdominal closure (TAC) that allows for subsequent interventions.

Forced closure of the abdominal cavity can lead to an increase in intra-abdominal pressure (IAP) in the patient, potentially resulting in the previously mentioned ACS, which is a condition characterized by increased pressure in the abdominal cavity. This leads to abnormal venous return and impaired blood supply or perfusion of abdominal and retroperitoneal organs such as the kidneys, causing a decrease in perfusion that can result in organ failure and patient death.

Over the years, various strategies have been developed to mitigate intra-abdominal hypertension and prevent its progression to ACS. Monitoring of IAP has been key for early detection of ACS. Early detection enables rapid treatment, initially with minimally invasive procedures, which, if ineffective, requires surgical decompression leaving the abdomen open (OA).

Currently, there are known methods for treating ACS, such as vacuum-assisted closure (VAC), specifically the "ABThera" system, which performs negative pressure therapy on the open abdomen (NPT OA). This method is indicated for the temporary management of an open abdomen when primary closure is not possible and/or when multiple abdominal reinterventions are necessary.

Among the numerous therapeutic benefits provided by this system, it is noteworthy that it delivers homogeneous tension, reduces lateralization and retraction of the abdominal wall, and is useful for achieving primary closure. However, as previously mentioned, primary closure is not always feasible, making it important to obtain the safest possible closure of the abdominal wall.

Additionally, a large number of complications can arise during the treatment of a patient with an open abdomen, related to the intra-abdominal issues that led to this management. Planning for a safe closure begins immediately after the abdomen is opened, as definitive closure of the abdominal wall is difficult to achieve in a significant percentage of patients, many of whom end up with a ventral hernia or require a secondary intention closure of the abdominal wall, which is very difficult and costly to treat definitively.

To address these issues and avoid the need to leave a large abdominal defect for delayed closure, techniques such as polypropylene mesh wall traction are used. However, this method has the disadvantage that it is sutured to aponeurosis, where the sutures compromise 2 cm of aponeurosis on each side, making definitive closure more difficult. Moreover, the tension cannot be adjusted during therapy without operating on the patient.

In the prior art, there are commercial devices like the "ABRA-Abdominal Wall Closure System," developed by Southmedic. This system employs a set of tensioners or elastomers placed perpendicular to the incision that completely pass through the thickness of the patient's abdominal wall, from the skin to the inner part of the wall or peritoneum. These tensioners have retainers on the skin that progressively tighten over several days, gradually bringing the edges of the abdominal wall closer. The tension in the elastomers is adjusted from one to another until full closure is achieved, but this process is very labor-intensive during NPT.

The ABRA system is notably expensive and complex, and highly aggressive for the patient, as it generates a significant inflammatory response in the first 24-48 hours, as well as necrosis, ulcerations, and scarring on the abdominal wall, leaving sequelae on the skin. It also presents several issues when used concomitantly with NPT AA, as the system consists of a thick, non-fenestrated silicone membrane, meaning it does not have any discontinuity on its surface. This interferes with NPT in the open abdomen, preventing the transmission of NPT to the peritoneal surface and the drainage of fluids from the viscera via NPT aspiration, without achieving the treatment goals for the open abdomen. Furthermore, the elastomer traction system of the ABRA does not allow for good access to the abdominal cavity during reinterventions to address the cause of the open abdomen and to proceed with the dressing change.

The patent document WO2018109253, owned by this applicant, discloses a previous development by the inventor, consisting of a dynamic wall traction device for the progressive closure of an open abdomen, which aimed to address the aforementioned drawbacks.

This device comprises two sheets or laminar bodies made of fenestrated material that are attached to the abdominal wall. Each body has a plurality of through-holes, through which traction wires allow the pulling of both bodies, enabling the progressive closure of the abdominal cavity. Although this device improves the passage of fluids with NPT aspiration in the open abdomen, it is still necessary to simplify and optimize the progressive closure of the open abdomen, as well as the use of NPT.

The document US2008306496A1 refers to devices and methods for closing the abdomen between surgical procedures and maintaining normal physiological tension in the fascia to prevent improper retraction. The document proposes using several closure devices, each of which includes a pair of "needled carabiner" elements connected to the ends of specific tensioning means, such as an elastic band with a specific tension.

This system, like the ABRA, is notably complex as it requires four to eight closure devices depending on the length of the abdominal incision, complicating the necessary reinterventions in this type of therapy. Additionally, due to the rigid characteristics of the fixation rings, which are temporarily attached to the edges of the fascia during the opening of the abdominal wall, complications can arise from pressure on the adjacent intra-abdominal viscera, as the document does not describe its use with a NPT device for the open abdomen, which utilizes a visceral protective sheet.

The document EP1982656A1 discloses systems and methods for approximating tissues by attaching anchors that are difficult to secure without a substantial volume of tissue and by performing a broad exposure of the anterior aponeurosis of the abdomen on opposite sides of a wound and coupling the anchors together.

This procedure of exposing the aponeurosis or fascia increases morbidity without offering a clear advantage. The intended use for the progressive closure of an open abdomen associated with NPT is not indicated, and like the ABRA system or the device described in US2008306496A1, it is a rather complex system that complicates reinterventions within the abdominal cavity as well as the use of NPT.

Finally, document WO2014125148A1 discloses a kit of specially designed elements for performing a closure procedure after abdominal surgery in patients where direct primary suturing of the laparotomy cannot be performed.

This kit includes elastic strips that are attached to anchors affixed to the skin of the wound, meaning the closure does not allow for effective traction of the entire abdominal wall, and the elastic strips may adhere to the adhesive sheet of the NPT, preventing it from fulfilling its purpose.

Therefore, there is a need for a device or system that enables dynamic traction of the wall in OA wounds and that overcomes the drawbacks posed by the current solutions in the state of the art.

### DESCRIPTION OF THE INVENTION

The object of the invention is a dynamic abdominal wall traction system for open abdomen wounds that avoids the previously mentioned drawbacks, and furthermore prevents the retraction of the aponeurosis from the outset of the open abdomen management, allowing a safe correction of the abdominal defect at the end of NPT OA.

With regard to what is disclosed in application WO2018109253 by the same applicant, and despite the fact that the device therein improved the passage of fluids with the aspiration of NPT OA, the system proposed here simplifies and optimizes the progressive closure of the open abdomen (OA), as well as the use of NPT.

This system is intended to be positioned in an open abdomen and essentially comprises:
- At least two retaining elements;
- Elastomeric rings, through which traction wires are inserted; and
- A traction element linked to the traction wires.

The retaining elements are designed to be sutured near two opposite edges of the open abdomen, more specifically at the internal or peritoneal edge of the abdominal wall. In the preferred embodiment of the system, each retaining element is positioned on the inner wall of the wound at a distance of between 3 and 4 centimeters from the respective upper aponeurotic edge of the abdominal wall.

Each retaining element is formed by an essentially flat and elongated piece that includes an upper sector, a lower sector, and transverse notches defined in two opposing zones of the peripheral edge, which separate the two sectors. In the upper sector, there is a through hole at the top and a through slot at the bottom, which extends from the through hole and continues towards the lower sector.

The suture that links the retaining element to the edge of the abdominal wall, whether thread, tape, or similar, is inserted through the through slot and then slid into the through hole, which retains the suture at the point where its loop is located. This prevents lateral movement of the retaining element, ensuring that it remains correctly linked to the inside of the abdominal wall.

The elastomeric rings are positioned in opposing pairs on two opposite edges of the open abdomen. Each elastomeric ring is also linked to a respective retaining element by a suture thread.

In this way, the elastomeric rings are not directly linked to the aponeurotic edge of the open abdomen, but instead are connected to the anatomy through the respective retaining elements to which they are linked. The suture linking each ring to its retaining element passes through the aponeurosis and exits through an upper edge of the aponeurosis to join with the elastomeric ring, thus preventing interference with the aponeurotic edge and avoiding damage or injury.

This represents one of the main advantages of the system. The retaining elements allow the traction for closing the open abdomen to be performed preferably from the inner (peritoneal) edge of the abdominal wall, pulling the aponeurotic/muscular structure of the abdominal wall towards the midline. Therefore, an "upward" traction of the aponeurosis is generated, reaching the midline of the open abdomen in an appropriate and anatomical manner through the vectors, by tensioning or pulling the entire abdominal wall structure.

The tension-traction vectors thus generated are more favorable than those of the ABRA system, where the wall is pulled from the skin to the peritoneum "downward," and the traction is not pulling or preventing the retraction of the wall in an adequate manner. In addition to the previously described complications in the subcutaneous tissue and skin, by being positioned 3 or 4 centimeters from the aponeurotic edge, the system avoids damaging the aponeurotic edge, leaving it intact and well-vascularized for primary closure at the end of the open abdomen management. This is not the case with the mesh that is sutured to the aponeurotic edge, nor with the ABRA system, which, by pulling the entire wall, may interfere with vascularization.

Finally, the traction wires or tapes, passing between the rings and interwoven in a zigzag pattern between the opposing elastomeric rings, allow for the application of a variable traction force that progressively approximates both sides of the open abdomen to achieve its closure, pulling the rings and, consequently, the aponeurotic edges to which they are attached.

Each of these traction wires comprises a free end linked to a single traction element, which enables the application of a variable and controllable traction force. In the preferred embodiment of the system, the traction element consists of a manually operable tourniquet.

In an alternative embodiment, the system incorporates a plurality of additional elastomeric rings, linked to each of the main elastomeric rings, with different diametric dimensions and tension. These additional rings allow for a gradual increase in the degree of tension and a decrease in the system's elasticity by exerting a force from the traction element.

The dynamic wall traction system for open abdomen wounds provides a simple solution that overcomes the drawbacks of the current state of the art.

### DESCRIPTION OF THE DRAWINGS

To complement the description and to aid in a better understanding of the features of the invention, in accordance with a preferred practical embodiment of the same, a set of drawings is included as an integral part of this description, in which the following is shown in an illustrative but non-limiting manner:
Figure 1.- Shows a perspective view of the dynamic traction system applied to an open abdomen.
Figure 2.- Shows a perspective view of a retaining element of the system.
Figure 3.- Shows schematically the connection between the rings and the retaining elements of the system, and their arrangement in an open abdomen.

### PREFERRED EMBODIMENT OF THE INVENTION

The following provides, with the help of the aforementioned figures, a detailed explanation of a preferred embodiment of the subject of the present invention. The dynamic wall traction system for open abdomen wounds described is intended to be temporarily sutured to an open abdomen (hereinafter also referred to as OA) wound to contribute to a progressive and controlled closure.

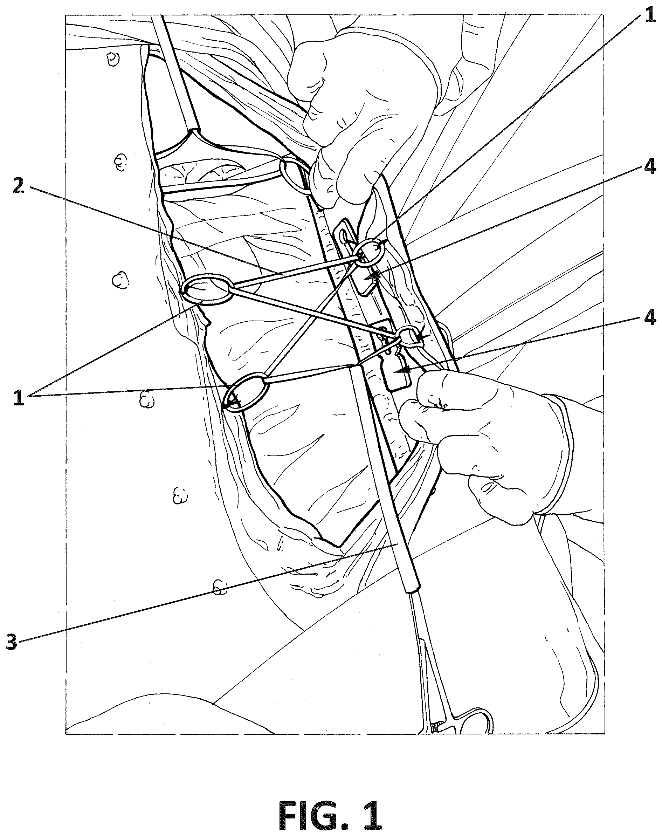

To achieve this, the system essentially comprises elastomeric rings (1) arranged on two opposite edges of the OA, traction wires (2) passing between the rings (1), a traction element (3) connectable to the traction wires (2), and retaining elements (4), which are in turn connectable both to the rings (1) and to the internal edges of the OA. Figure 1 illustrates the system applied to an OA.

The rings (1) are arranged in pairs facing each other near the external or aponeurotic edges of the OA. Each ring (1) is connected to a respective retaining element (4) by a suture thread.

The traction wires (2), passing through the inside of the rings (1) and interwoven in a zigzag pattern between the opposing pairs of rings (1), allow for the application of a variable traction force that brings the rings (1) closer together, and consequently the edges, to contribute to the progressive closure of the open abdomen.

Each traction wire (2) has a free end that can be linked to the traction element (3). This traction element (3) allows for the application of a variable and controllable traction force on the traction wires (2), and consequently on the rings (1). In the preferred embodiment shown, the traction element (3) consists of a manually operable tourniquet. In alternative embodiments of the system, the traction element consists of a micrometric closure.

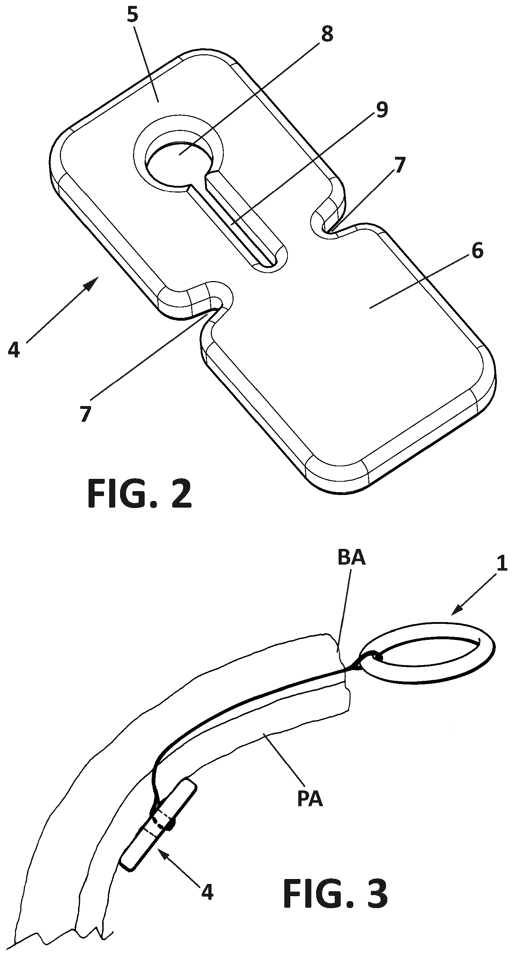

Figure 2 shows in detail one of the retaining elements (4) used in the system. As previously mentioned, each retaining element (4) is sutured near the two opposing edges of the open abdomen, specifically at the internal or peritoneal edge of the abdominal wall (PE). In the preferred embodiment of the system, each retaining element (4) is placed at a distance of between 3 and 4 centimeters from the respective aponeurotic edge (AE) of the abdominal wall (AW), as seen in Figures 1 and 3.

Each retaining element (4) is formed from an essentially flat and elongated piece, shaped like a beveled-edged plate, which includes an upper sector (5), a lower sector (6), and transverse notches (7) defined at two opposite areas of the peripheral edge, separating the two sectors (5, 6). The upper sector (5) contains an upper through hole (8) and a lower through slot (9), which starts from the through hole (8) and extends toward the lower sector (6).

The suture linking the retaining element (4) to the inside of the abdominal wall (AW) is inserted through the lower through slot (9) and is then slid up to the through hole (8), which holds the suture by its looped end. This prevents lateral displacement of the retaining element (4), ensuring it is properly attached to the abdominal wall (AW).

This same suture of the retaining element (4) passes through the aponeurosis and exits at the upper edge of the aponeurosis to connect with the corresponding ring (1). This design avoids a direct connection between the ring (1) and the aponeurotic edge (AE), preventing any damage or injury. Figure 3 schematically illustrates this connection between the ring (1) and retaining element (4).

In an alternative embodiment not shown in the attached figures, the system incorporates a plurality of additional elastomeric rings, each linked to one of the main elastomeric rings (1), with different diametrical dimensions and tension levels. The additional rings allow for progressively increasing the tension and reducing the system's elasticity by exerting a force from the traction element (3).

## Claims

1. Dynamic abdominal wall traction system for open abdomen wounds, temporarily attachable to an open abdomen (OA) wound for progressive and controlled closure, comprising:
- elastomeric rings (1), attachable near the aponeurotic edges of the OA, and arranged in pairs facing each other on opposite edges of the OA;
- traction wires (2), interwoven in a zigzag pattern between the pairs of facing rings (1) and passing through their interior, where each traction wire (2) has a free end; and
- a traction element (3), attachable to the free ends of the traction wires (2) to apply a variable and controllable traction force on the traction wires (2) and the rings (1);
being the dynamic traction system **characterized in that**:
- it additionally comprises retaining elements (4), attachable by suturing to both an internal peritoneal edge of the abdominal wall (AW) and each of the rings (1); and **in that**
- the system comprises the same number of rings (1) as retaining elements (4), such that the connection between the rings (1) and the OA wound is indirect, with the mediation of the suturing connection to the respective retaining elements (4).

2. Dynamic abdominal wall traction system according to claim 1, wherein each retaining element (4) is formed by an essentially flat and elongated piece, in the shape of a beveled-edge plate comprising:
- an upper sector (5) with an upper through hole (8) and a lower through slot (9);
- a lower sector (6); and
- transverse notches (7), defined in two opposite areas of the peripheral edge of the plate, which separate both sectors (5,6).

3. Dynamic abdominal wall traction system according to any of the previous claims, further comprising a plurality of additional elastomeric rings, linked to each of the main elastomeric rings (1).

4. Dynamic abdominal wall traction system according to any of claims 1-3, wherein the traction element (3) is a manually operable tourniquet.

5. Dynamic abdominal wall traction system according to any of claims 1-3, wherein the traction element (3) is a micrometric closure.
